Europäisches Patentamt

European Patent Office

Office européen des brevets

(19)

(11) Numéro de publication: **0 206 870**
**B1**

(12) **FASCICULE DE BREVET EUROPEEN**

(45) Date de publication du fascicule du brevet:
**01.02.89**

(21) Numéro de dépôt: **86401135.8**

(22) Date de dépôt: **29.05.86**

(51) Int. Cl.⁴: **C09C 1/00**, C01F 17/00,
A61K 7/021

(54) Pigments à base de cérium et de praséodyme, leur procédé de préparation et leurs applications.

(30) Priorité: **07.06.85 FR 8508611**

(43) Date de publication de la demande:
**30.12.86 Bulletin 86/52**

(45) Mention de la délivrance du brevet:
**01.02.89 Bulletin 89/5**

(84) Etats contractants désignés:
**AT BE CH DE FR GB IT LI LU NL SE**

(56) Documents cités:
**DE-C- 117 665**
**FR-A- 1 257 078**
**FR-A- 1 540 212**
**FR-A- 2 249 032**

(73) Titulaire: **RHONE-POULENC CHIMIE, 25, quai Paul Doumer, F-92408 Courbevoie Cédex(FR)**

(72) Inventeur: **Maestro, Patrick, 27, rue Honoré de Balzac, F-95470 - Fosses(FR)**
Inventeur: **Tastu, Francis, Lotissement Les Tamaris Cour des Alouettes No. 1, F-17137 Nieul Sur Mer(FR)**

(74) Mandataire: **Dutruc-Rosset, Marie-Claude et al, RHONE-POULENC INTERSERVICES Service Brevets Chimie 25, Quai Paul Doumer, F-92408 Courbevoie Cédex(FR)**

ACTORUM AG

## Description

La présente invention concerne des pigments à base de cérium et de praséodyme et plus particulièrement ceux à composante rouge. Elle vise également leurs procédés de préparation ainsi que leurs applications, en particulier, dans l'industrie de la céramique.

Les pigments sont largement utilisés dans de nombreuses industries notamment dans les peintures et lasures, les matières plastiques et l'industrie de la céramique. Cette dernière demande des pigments qui conservent leur teinte lorsqu'ils sont introduits dans l'agent vitrifiant: l'émail.

Parmi les couleurs du spectre visible, on n'est pas parvenu jusqu'à présent, à obtenir des pigments rouges de teinte soutenue et stables à haute température. En effet, les corps colorants utilisés actuellement qui sont les uranates de sodium, et des dérivés du sulfure de cadmium ne peuvent être utilisés qu'à basse température c'est-à-dire à une température inférieure à 850°C. Par ailleurs, ils présentent un autre inconvénient non négligeable, qui réside dans leur grande toxicité ce qui pose toujours des problèmes au niveau de leur mise en œuvre.

Dans le brevet FR-A 1 257 078 on a décrit un pigment rouge à base de cérium et de praséodyme qui est obtenu par calcination à une température de 1000 à 1500°C d'un mélange d'oxyde de cérium, d'oxyde de praséodyme et éventuellement de chlorure de cérium et de chlorure de praséodyme. Ce mélange peut être additionné de chlorures alcalins et d'oxydes de titane, de silice ou d'alumine. Il s'avère que la couleur de la poudre ainsi obtenue n'est pas totalement stable lorsqu'elle est incorporée dans des frittes classiques. L'analyse cristallographique montre qu'elle est constituée non pas d'un seul oxyde mais d'un mélange d'un oxyde mixte de cérium et de praséodyme, d'oxyde de cérium et d'oxyde de praséodyme.

La Demanderesse a découvert que par calcination de co-précipités d'hydroxydes ou de sels insolubles de cérium et de praséodyme, il était possible d'obtenir un nouveau pigment caractérisé par une structure cristallographique unique et par une coloration plus intense que celle du pigment décrit dans FR-A 1 257 078.

La présente invention a ainsi pour objet des pigments répondant à la formule:

$$Ce_{1-x-y} PR_x A_y O_z \quad (I)$$

dans laquelle:

A est au moins un élément qui peut être:

— un élément terre rare $A_1$ choisi dans le groupe constitué par les lanthanides et l'yttrium et/ou

— un élément de transition $A_2$ choisi dans le groupe formé par les éléments appartenant aux colonnes 1b, 2b, 3b, 4b, 5b, 6b, 7b, 8 de la classification périodique telle que définie dans "Handbook of Chemistry and Physics B-4 (57ème édition)".

— x étant un nombre tel que $0,001 \leq x \leq 0,10$
— y étant un nombre tel que $0 \leq y \leq 0,1$ et
— z étant égal à

$$\frac{4 - (4-n)y}{2}$$

— n étant le degré d'oxydation de A
ayant une structure cristallographique unique isotype de celle de $CeO_2$ et présentant un seuil de réflectance d'au moins 580 nm.

Les pigments préférés selon l'invention sont ceux qui répondent à la formule (I) dans laquelle $A_1$ est choisi parmi les éléments terres rares suivants: terbium, néodyme, erbium, dysprosium, europium et $A_2$ est choisi parmi les éléments de transition suivants: titane, vanadium, chrome, manganèse, cuivre, fer, cobalt, nickel et molybdène. $A_2$ est de préférence le cuivre.

Dans l'exposé qui suit de l'invention on entend respectivement par "élément $A_1$" ou "élément $A_2$", un ou plusieurs éléments $A_1$ ou $A_2$.

Dans la formule (I), x est de préférence inférieur ou égal à 0,06. De plus y est avantageusement inférieur ou égal à 0,05.

Les pigments selon l'invention sont constitués par une solution solide d'oxyde de praséodyme et éventuellement d'un oxyde de l'élément A dans l'oxyde de cérium, c'est-à-dire que le praséodyme et éventuellement l'élément A sont présents dans le réseau cristallin de l'oxyde de cérium, sans constituer de phases cristallines distinctes.

La stabilisation du praséodyme tétravalent dans la matrice de l'oxyde de cérium permet l'obtention de couleur rouge liée à un spectre de bande dont le seuil de réflectance est situé à au moins 580 nm et dans le cas des pigments préférés à au moins 590 nm: le seuil étant déterminé à partir du spectre de réflexion diffuse obtenu à l'aide d'une sphère intégratrice.

La présente invention a également pour objet le procédé de préparation des pigments de formule (I) qui peut être réalisé suivant trois variantes d'exécution selon que l'élément A est un élément terre rare $A_1$

EP 0 206 87u B1

ou un élément de transition $A_2$ ou un mélange des deux.

Le procédé de l'invention est caractérisé par le fait qu'il consiste:

a) à effectuer une co-précipitation de composés de cérium, de praséodyme et éventuellement de l'élément $A_1$ à partir d'une solution aqueuse de sels solubles des éléments précités,

b) à séparer le co-précipité obtenu,

c) à ajouter éventuellement un composé de l'élément $A_1$ et/ou un composé de l'élément $A_2$ sous forme solide, au co-précipité obtenu,

d) à calciner le mélange obtenu à une température d'au moins 800°C.

Dans la première étape du procédé de l'invention, on co-précipite le cérium, le praséodyme et éventuellement l'élément $A_1$.

Les solutions aqueuses de sels solubles que l'on utilise au départ peuvent être notamment des solutions de nitrates, de chlorures et/ou de sulfates.

Le sel de cérium peut être un sel de cérium à l'état céreux ou à l'état cérique. En outre il est possible d'utiliser un sel céreux et de l'oxyder ensuite dans le milieu réactionnel à l'état cérique avec un agent oxydant. Parmi les agents oxydants pouvant convenir, on peut citer notamment des solutions de perchlorate, de chlorate, d'hypochlorite, de persulfate de sodium, de potassium ou d'ammonium, d'eau oxygénée ou l'air, l'oxygène, l'ozone. On peut également oxyder le cérium par voie électrochimique. On utilise de préférence l'eau oxygénée. La proportion d'agent oxydant par rapport au sel céreux à oxyder peut varier dans de larges limites. Elle est en général supérieure à la stœchiométrie et correspond, de préférence, à un excès compris entre 5 et 40%.

Le sel de cérium est choisi de telle sorte qu'il ne contienne pas d'impuretés qui puissent se retrouver dans le produit calciné. Il peut être avantageux de mettre en jeu un sel de cérium présentant un degré de pureté supérieur à 99%.

La concentration dans la solution, du sel de cérium n'est pas un facteur critique selon l'invention et elle peut varier dans de larges limites; une concentration comprise entre 0,2 et 4 moles par litre est préférée.

Le sel de praséodyme et éventuellement de l'élément $A_1$ qui peuvent être anhydres ou hydratés, sont choisis préférentiellement d'une grande pureté comme le sel de cérium.

La concentration dans la solution, du sel de praséodyme et éventuellement de celle de l'élément $A_1$ n'est pas critique et peut également varier de préférence entre 0,2 et 4 moles par litre.

On effectue le mélange, dans un ordre quelconque, les différents composés précités dont les proportions sont telles que l'on obtienne les pourcentages pondéraux définis dans la formule (I).

Comme composés de cérium, de praséodyme et de l'élément $A_1$ que l'on peut précipiter, on peut citer notamment les hydroxydes, les oxalates et les carbonates.

La co-précipitation des hydroxydes peut être effectuée par mélange d'une solution aqueuse des sels solubles avec une solution basique.

La solution basique mise en œuvre peut être notamment une solution aqueuse d'ammoniaque ou d'hydroxyde de sodium, de potasse. On met en œuvre de préférence une solution d'ammoniaque. La normalité de la solution basique mise en œuvre n'est pas un facteur critique selon l'invention, elle peut varier dans de larges limites, elle sera toutefois avantageusement comprise entre 1 et 5 N, de préférence 2 à 3 N. Il peut être particulièrement avantageux de régler le pH dans ces limites à une valeur constante à $\pm$ 0,1 unité de pH.

La proportion entre la solution basique et la solution de sels solubles de cérium, de praséodyme et éventuellement de l'élément $A_1$ doit être telle que le nombre d'équivalents basiques soit supérieur ou égal au nombre d'équivalents cérium, praséodyme et élément $A_1$. Il peut être avantageux de mettre en œuvre un excès supérieur à 5% d'équivalents basiques. Le pH du milieu réactionnel, peut varier entre 6 et environ 8,5. Il est avantageusement compris entre 7,5 et 8,5.

La température du milieu réactionnel doit être comprise, de préférence, entre 10 et 95°C et, plus particulièrement, entre 50 et 70°C. Le temps de séjour du mélange dans le milieu réactionnel n'est pas un facteur critique selon l'invention et peut varier dans de larges limites; généralement, on choisira des temps de séjour compris entre 30 minutes et 2 heures.

On obtient un co-précipité mixte d'hydroxydes de cérium, de praséodyme contenant éventuellement un élément d'une autre terre rare ($A_1$).

Un procédé préféré de l'invention est la co-précipitation oxalique qui peut être réalisée éventuellement en présence d'un élément $A_1$.

Les solutions de sels de cérium, praséodyme et d'élément(s) $A_1$ répondent aux caractéristiques précitées.

Pour ce qui est de l'agent précipitant, on peut faire appel à l'acide oxalique ou à ses sels de préférence ammonium, sous forme anhydre ou hydratée.

On peut le mettre en œuvre sous forme cristallisée ou sous forme de solution aqueuse.

Dans ce cas, la concentration exprimée en acide oxalique peut varier entre 0,8 et 3 moles/litre de préférence 0,3 à 1 mole/litre.

La proportion entre la solution oxalique et la solution de sels solubles de cérium, de praséodyme et éventuellement de l'élément $A_1$ est telle que le nombre d'équivalents oxalique soit égal ou supérieur au nombre d'équivalents cérium, praséodyme et élément $A_1$. On peut utiliser un excès représentant jusqu'à 50% de la stœchiomètrie.

La co-précipitation est effectuée à une température comprise entre 20 et 100°C, préférentiellement 80 à 90°C et à un pH de 1 à 3.

Comme dans la précipitation des hydroxydes, le temps de séjour n'est pas un facteur critique et les conditions décrites restent valables.

On obtient un co-précipité d'oxalates de cérium, de praséodyme et éventuellement d'un élément $A_1$.

Les co-précipités d'hydroxydes et d'oxalates préparés ci-dessus peuvent être ou non soumis à un mûrissement selon la morphologie des particules que l'on désire obtenir.

La masse réactionnelle peut être mûrie pendant un certain temps à une température comprise entre environ 10 et 95°C et, de préférence, entre 50 et 80°C avant l'opération de filtration. Dans ce cas, le temps de mûrissement n'est pas un facteur critique selon l'invention et peut varier dans de larges limites; toutefois, un temps pouvant aller jusqu'à environ 2 heures est généralement satisfaisant.

La seconde étape du procédé consiste à séparer le co-précipité de la suspension obtenue. Cette séparation peut être effectuée selon les techniques classiques de séparation liquide/solide telles que décantation, essorage, filtration et/ou centrifugation.

Selon une variante du procédé de l'invention, le co-précipité séparé peut être ensuite lavé avec de l'eau. Après ce lavage, la teneur en eau du produit est comprise entre 20 et 80% en poids et, généralement, entre 30 et 50%.

Le produit obtenu après séparation et éventuellement lavage peut ensuite être séché. La température de séchage est comprise de préférence entre 100 et 550°C; le temps de séchage variant de préférence entre 30 minutes et 2 heures.

Le produit ainsi obtenu, lorsque le co-précipité est un précipité d'oxalates est avantageusement soumis à une pré-calcination afin de décomposer les oxalates.

Cette opération peut être effectuée à une température de 350 à 700°C pendant une durée de 30 minutes à 2 heures.

Une variante d'exécution du procédé de préparation des composés de formule (I) contenant un élément $A_1$ consiste à effectuer non pas une co-précipitation cérium, praséodyme et élément $A_1$ mais à réaliser d'abord la préparation d'un co-précipité de cérium et de praséodyme puis à le mélanger avec un composé de l'élément $A_1$ sous forme solide, avant l'opération de calcination.

A titre de composés de l'élément $A_1$, on peut citer les oxydes de l'élément $A_1$ ou tout sel de cet élément décomposable dans les conditions de calcination, par exemple, nitrates, chlorures, sulfates, carbonates.

On effectue le mélange intime du co-précipité séparé cérium, praséodyme et du composé de l'élément $A_1$ soit avant ou après séchage, soit avant ou après pré-calcination selon que ces étapes aient lieu ou non.

On peut également ajouter à ce stade, un fondant dont la nature est précisée ci-après.

Un autre mode de réalisation de l'invention consiste à préparer des composés de formule (I) faisant intervenir un élément $A_2$ selon un procédé qui consiste à mélanger, avant calcination, un composé de l'élément $A_2$ sous forme solide au co-précipité séparé à l'étape précédente.

Le composé de l'élément $A_2$ peut être les oxydes de l'élément $A_2$ ou tout sel de cet élément décomposable.

Le mélange intime du co-précipité et du composé de l'élément $A_2$ est effectué avant calcination dans les mêmes conditions décrites pour le composé de l'élément $A_1$ si bien qu'il est également possible de les mettre en œuvre ensemble si le composé de formule (I) les renferme simultanément.

Avant la calcination, on peut également ajouter un fondant ou agent minéralisateur qui permet d'abaisser la température de calcination ou à même température de calcination d'améliorer la réactivité.

En tant que fondant, on utilise des composés qui présentent un point de fusion nettement inférieur à la température de formation de la solution solide des oxydes des métaux intervenant dans la formule (I), qui soient inertes vis-à-vis de la structure cristalline recherchée et qui soient susceptibles d'être facilement éliminés soit par vaporisation au cours de la réaction, soit par un lavage consécutif, généralement à l'eau.

Comme exemples de fondants, on peut citer d'une manière non limitative, les différents fluorures, chlorures, bromures et nitrates des métaux alcalins tels que le lithium, le potassium ou le sodium ou bien du borate de sodium. Ils peuvent être mis en œuvre seuls ou en mélange. D'une manière préférentielle, on utilise le fluorure de sodium ou de lithium, le chlorure de sodium ou de lithium ou leur mélange.

La quantitié de fondant est calculée par rapport au poids du co-précipité exprimé en oxydes et lorsqu'il y a une pré-calcination par rapport au poids du mélange pré-calciné des composés des différents éléments. Elle peut varier sans inconvénient, dans de larges limites mais représente plus préférentiellement de 1 à 10% en poids dudit mélange.

Dans le cas de la mise en œuvre d'un fondant, il est souhaitable de procéder au mélange intime du fondant et du co-précipité, après avoir séché ce dernier et de préférence soumis à une pré-calcination.

On réalise ensuite l'étape de calcination à une température de 800 à 1500°C et de préférence de 1000 à 1400°C. La calcination est effectuée généralement à l'air pendant une durée qui peut varier le plus souvent entre 10 et 20 heures. Après refroidissement le fondant peut être éliminé par lavage à l'eau, le produit calciné étant ensuite séché.

Le produit ainsi obtenu peut être ensuite broyé à une taille de particules de 0,1 à 10 μm, pour qu'il puisse être utilisé efficacement comme pigment.

Le procédé de l'invention peut être aisément mis en œuvre en continu.

Il peut être mis en œuvre dans un appareillage classique. L'étape de co-précipitation peut être effectuée dans un réacteur équipé d'un dispositif de chauffage thermorégulé, des moyens usuels de contrôle de réaction (thermomètre), des moyens d'agitation (agitation à ancre ou à hélice), des moyens d'introduction des réactifs et d'une unité de régulation du pH placée à la sortie du réacteur.

La séparation du co-précipité peut être effectuée par exemple sur un dispositif continu de filtration par exemple, un filtre rotatif type Vernay ou un filtre à bande.

Les opérations de séchage et de calcination peuvent être effectuées dans deux appareils distincts ou s'enchaîner dans un seul appareil du type four rotatif. On utilise de préférence un four rotatif légèrement incliné permettant ainsi la circulation de la matière et assurant dans sa première partie, le séchage du produit et dans sa deuxième partie, sa calcination en raison d'un gradient de température plus élevé dû à la proximité de la flamme que l'on alimentera, de préférence avec un gaz naturel.

Après calcination, le produit obtenu peut être soumis à des opérations de broyage dans un microniseur.

Les pigments selon l'invention peuvent être utilisés dans des applications aussi variées que les peintures et lasures, les matières plastiques et notamment les revêtements de sol, l'industrie du papier, des encres d'imprimerie, les textiles, etc.

Un domaine d'application privilégié des pigments selon l'invention est l'industrie de la céramique, en raison de leur stabilité aux hautes températures.

Il est possible de les mettre en oeuvre dans un domaine de température de 800°C à 1250°C sur différents supports tels que la terre cuite, la faience, le grès, la porcelaine, avec différentes glaçures. Par glaçure, on entend la mince couche de verre qui recouvre le support et qui prend le nom de "couverte" lorsqu'elle est transparente et alcaline et recouvre les pâtes à grès et porcelaine. La glaçure est obtenue par chauffage et refroidissement de la fritte qui peut être composée de nombreux oxydes tels que $SiO_2$, $Al_2O_3$, $Na_2O$, $K_2O$, $PbO$, $CaO$, $BaO$, $MgO$, $ZnO$, $SrO$, $Sb_2O_3$, $Li_2O$, $B_2O_3$. L'oxyde le plus couramment rencontré est $SiO_2$, les autres oxydes pouvant être considérés comme des fondants qui ont pour effet d'abaisser la température de fusion de la silice.

Les pigments de l'invention peuvent être utilisés selon n'importe quelle technique d'émaillage, qu'il soit réalisé manuellement au pinceau, au trempé ou par pulvérisation ou qu'il soit réalisé industriellement sur des machines qui reprennent ces procédés traditionnels: émaillage par rideau, en trempé ou par pulvérisation.

Les pigments de l'invention peuvent être utilisés également dans le domaine de la cosmétique et notamment dans les vernis à ongles et dans les fards tels que rouges à lèvres, fards secs, fards gras ou fonds de teint.

Ils peuvent donc être mis en œuvre dans les vernis à ongles qui contiennent généralement:
– un agent filmogène à base de nitrocellulose,
– une résine, résine dammer naturelle ou résine synthétique du type formaldéhyde-sulfamide, résine polystyrène, résine polyvinylique, etc.,
– un plastifiant par exemple, le phtalate de diéthyle, le phtalate de dibutyle, le phtalate de dioctyle, le tricrésylphosphate, le stéarate de n-butyle, le diacétate de résorcine ou leur mélange,
– un dissolvant tel que l'alcool éthylique, isopropylique, butylique, isobutylique, l'acétate d'éthyle, l'acétate de butyle ou le plus souvent le mélange de ces solvants,
– un diluant, notamment le toluène ou le xylène,
– éventuellement d'autres additifs, parfum ou produit nacrant (flocons de mica enrobés d'oxychlorure de bismuth ou de bioxyde de titane).

On donne ci-après un exemple de composition-type:
– de 10 à 15% en poids de nitrocellulose
– de 10 à 15% en poids de résine
– de 3 à 5% en poids de plastifiant(s)
– de 3 à 5% en poids de pigment(s)
– q.s.p. 100% en poids de solvant(s)

Généralement les pigments sont broyés dans une masse plastique constituée de nitrocellulose et de plastifiant(s) qui est ensuite mise en solution dans le(s) solvant(s).

Une autre application des pigments de l'invention est celle des rouges à lèvres.

Les pigments sont mis en oeuvre le plus souvent à raison d'une concentration pondérale de 5 à 15% exprimée par rapport à la formulation totale qui renferme:
– un excipient formé d'un mélange de divers corps pour assurer la consistance: cire d'abeille, cire de carnauba, ozocérites, paraffine, cires synthétiques ou leur mélange et d'un excipient mou permettant d'ajuster la consistance tel que le beurre de cacao, la vaseline, les huiles blanches hydrogénées par exemple, l'huile de palme, d'arachide ou de ricin,
– divers additifs notamment un parfum ou arome et le myristate d'isopropyle ou le palmitate d'isopropyle qui donne du glissant,

– un solvant intermédiaire pour mettre en suspension le pigment dans la phase lipophile qui peut être l'huile de ricin ou un glycol comme le polyoxyéthylèneglycol 400 ou des esters d'acides gras: monoricinoléate de propylène glycol, myristate d'isopropyle, palmitate d'isopropyle, stéarate de butyle.

On donne, ci-après, à titre illustratif, un exemple de composition de rouge à lèvres:

| | |
|---|---|
| – ozocérite | 4% en poids |
| – cire de carnauba | 2% en poids |
| – cire de candelilla | 8% en poids |
| – mélange de diesters de propylèneglycol d'acides caprylique et caprique | 31% en poids |
| – huile de ricin | 24% en poids |
| – huile de vaseline | q.s.p. 100% en poids |
| – para-oxybenzoate de propyle | 0,1% en poids |
| – tertiobutyl hydroxyanisole | 0,1% en poids |
| – pigment coloré | 2,5% en poids |
| – oxyde de titane | 0,5% en poids |
| – huile de ricin | 15,0% en poids |
| – parfum | 0,50% en poids |

On fond le mélange des cires à une température supérieure à la température de fusion de la cire la plus élevée. Dans le mélange fondu, on ajoute le pigment en suspension dans le solvant puis le parfum. Enfin le mélange obtenu est coulé dans des moules.

Les fards à yeux et les fards à joues peuvent se présenter sous forme de fards secs ou de fards gras. La teneur en pigments dans de tels fards peut varier dans de larges limites de 5 à 20%.

Les fards secs sont des poudres (talc, carbonate de magnésium, stéarate de zinc) qui sont chargées en pigments et agglomérées soit avec de la méthylcellulose, soit avec des stéarates.

On donne, à titre d'exemple la composition d'un fard à paupières:

| | |
|---|---|
| – silicate d'aluminium et de magnésium (Veegum F) | 7% en poids |
| – talc | 50% en poids |
| – oxyde de zinc | 4% en poids |
| – stéarate de zinc | 11% en poids |
| – kaolin | 10% en poids |
| – pigment | 18% en poids |

Les pigments de l'invention peuvent également être employés dans les formulations de fonds de teint.

Les fonds de teint se présentent sous forme d'émulsion en général du type huile dans l'eau.

La phase lipophile comprend le plus souvent:

– un composant huileux tel que l'huile de vaseline, des esters d'acides gras et d'alcools éventuellement gras, par exemple, l'oléate d'oléyle, l'oléate de décyle, le stéarate d'octyle, l'adipate de di-n-butyle, le myristate d'isopropyle, le palmitate d'isopropyle, le stéarate d'isopropyle, les esters d'acides caprique et caprylique d'alcools gras saturés ayant de 12 à 18 atomes de carbone, une huile de silicone ou leur mélange entre eux,

– un agent émulsifiant du type anionique et/ou non-ionique et plus précisément les sels d'acides gras, stéarate de sodium, de potassium ou d'ammonium, palmitate de sodium; les esters de sorbitan et d'acides gras tels que, par exemple, l'acide laurique, l'acide palmitique, l'acide stéarique; les esters polyoxyéthylénés de sorbitan et d'acides gras contenant de 4 à 20 moles d'oxyde d'éthylène par mole d'ester; les alcools gras polyoxyéthylénés contenant de 2 à 23 moles d'oxyde d'éthylène par mole d'alcool, ledit alcool pouvant être notamment l'alcool laurique, l'alcool cétylique, l'alcool stéarylique, l'alcool oléylique; le mono- et distéarate de glycérol, le mono- et dioléate de glycérol; les acides gras poxyloxyéthylénés et en particulier le stéarate polyoxyéthyléné contenant de 18 à 100 moles d'oxyde d'éthylène par mole d'acide,

– un agent permettant d'ajuster la consistance qui peut être un alcool gras ou un acide gras et plus précisément l'alcool cétylique, l'alcool stéarylique, l'acide stéarique.

Quant à la phase hydrophile, elle est constituée d'eau, de préférence distillée et de divers additifs, notamment:

– un agent humectant qui peut être, par exemple, le propylèneglycol, le glycérol, le sorbitol,

– un agent conservateur et plus particulièrement l'o-phénylphénol et les acides suivants, leurs sels (Na, K, NH4) ou leurs esters ayant de 1 à 4 atomes de carbone: l'acide benzoïque, l'acide salicylique, l'acide sorbique, l'acide p-hydroxybenzoïque,
– un agent de stabilité notamment les dérivés cellulosiques dont la carboxyméthylcellulose et la gomme Xanthane.

Une illustration d'une formulation pour fonds de teint est donnée ci-après:

| | | |
|---|---|---|
| **– phase lipophile:** | | |
| – huile de vaseline | | 15% en poids |
| – mono- et distéarate de glycérol | | 4% en poids |
| – alcool cétylique | | 1% en poids |
| **– phase hydrophile:** | | |
| – eau distillée | q.s.p. | 100% en poids |
| – propylèneglycol | | 3% en poids |
| – para-oxybenzoate de méthyle | | 0,05% en poids |
| – para-oxybenzoate de propyle | | 0,1% en poids |
| **– pigment coloré** | | 1 à 10% en poids |
| **– oxyde de titane** | | 3% en poids |

La préparation des formulations de fonds de teint est conduite en dispersant d'abord le pigment dans la phase lipophile maintenue vers 60–80°C puis la phase hydrophile maintenue à une des températures comprises dans l'intervalle précité est ajoutée sous agitation et lentement dans la phase lipophile.

Dans l'exposé qui précède, il est donné des exemples de formulations destinées à la cosmétique dans lesquelles peuvent convenir les pigments de l'invention et il va sans dire que ces exemples de même que les différents composants cités ne présentent aucun caractère limitatif et ne sont donnés qu'à titre illustratif.

Les exemples suivants illustrent la présente invention. Parmi ces exemples, les exemples 1 à 4 et en partie les exemples 7 à 8 illustrent la préparation des pigments, les exemples 5 à 8 l'application à l'émaillage et l'exemple 9 l'application dans le domaine de la cosmétique.

Les pigments sont caractérisés par leur structure cristallographique et leur couleur.

La structure cristallographique est déterminée selon la méthode Debye et Scherrer en transmission: rayonnement monochromatique du molybdène.

La couleur est mesurée à l'aide d'un spectro-photocolorimètre utilisant une sphère intégratrice.

La mesure de la couleur est exprimée en système CIE Lab. Ce système de mesure de coordonnées trichromatiques (Principles of the Color Technology F. Billmeyer Jr et Max Saltzman 2ème édition Wiley), sous l'illuminant D 65, permet de situer les écarts de couleur et fait appel aux grandeurs suivantes L, a, b:

L donne une mesure de la réflectance (nuance claire/sombre) et varie de 100 (blanc) à 0 (noir)
a et b sont les valeurs des tendances colorées:
a positif = rouge
a négatif = vert
b positif = jaune
b négatif = bleu
L représente donc la variation du noir au blanc, a la variation du rouge au vert et b la variation du jaune au bleu.

EXEMPLE 1

On prépare une solution à partir de 395 g de nitrate de cérium $Ce(NO_3)_3$, $6H_2O$ et 20,4 g de nitrate de praséodyme $Pr(NO_3)_3$, $6H_2O$ que l'on dissout dans 1,5 litres d'eau. On chauffe la solution à environ 90°C et on ajoute sous agitation 284 g d'oxalate d'ammonium $(NH_4)_2C_2O_4$. On laisse agiter 10 minutes à la température de 90°C puis on laisse le co-précipité obtenu décanter une heure après arrêt du chauffage. On élimine la liqueur surnageante et on filtre le co-précipité qui est ensuite séché à 100°C et soumis à une première calcination à 560°C. On obtient ainsi 160 g d'un oxyde mixte de cérium et de praséodyme. Ce produit est calciné à 1400°C pendant 18 heures en présence de 3,2 g de fluorure de sodium NaF.

On obtient 160 g d'une solution solide de formule $Ce_{0,95} Pr_{0,05} O_2$ dont la structure cristallographique est isotype de celle de $CeO_2$ (JCPDS 4-0593). La substitution du cérium par le praséodyme est mise en évidence par la diminution du paramètre de maille: 5,407 Å pour $Ce_{0,95} Pr_{0,05} O_2$ au lieu de 5,411 Å pour $CeO_2$.

La coloration de la poudre est rouge bordeaux et est caractérisée par un seuil de réflectance de 590 nm et par les valeurs L = 35,7, a = 19,2 et b = 10,5 dans le système CIELab.

EXEMPLE COMPARATIF

On a préparé des pigments selon les exemples du brevet FR-A 1 257 078, et comparé les poudres obtenues à celle préparée selon l'exemple 1, en termes de couleur et de structure cristallographique.

Les poudres préparées selon le brevet FR-A 1 257 078 correspondent aux compositions suivantes (exprimées en gramme de produit).

|  | B1 | B2 | B3 | B4 |
|---|---|---|---|---|
| $CeO_2$ | 9,6 | 9,3 | 7,8 | 8,86 |
| $CeCl_3$ | – | – | – | 0,95 |
| $Pr_6O_{11}$ | 0,4 | 0,2 | 0,2 | 0,19 |
| $TiO_2$ | – | – | 1 | – |
| NaCl | – | – | 1 | – |
| LiCl | – | 0,5 | – | – |

Le tableau suivant donne la comparaison des colorations obtenues, en liaison avec la structure cristallographique.

| Echantillon | Couleur | L | a | b | Structure |
|---|---|---|---|---|---|
| Exemple 1 | rouge bordeaux | 35,7 | 19,2 | 10,5 | $Ce_{0,95}Pr_{0,05}O_2$ uniquement |
| B1 | brique | 50,9 | 18,6 | 16,1 | mélange |
| B2 | brique | 52,0 | 22,9 | 20,4 | $Ce_{0,95}Pr_{0,05}O_2$ + $Ce_2$ + «$PrO_2$» |
| B3 | marron clair | 56,6 | 7,4 | 9,7 | $Ce_{0,95}Pr_{0,05}O_2$ + $Ce_2$ + «$PrO_2$» |
| B4 | brique | 52,8 | 20,5 | 18,1 | $Ce_{0,95}Pr_{0,05}O_2$ + $Ce_2$ + «$PrO_2$» |

Les différences observées montrent que le pigment de l'exemple 1 permet une coloration mieux définie dans le rouge que l'on attribue à une plus grande pureté de la structure cristallographique, le praséodyme, élément responsable de la coloration étant totalement inséré dans la maille cristalline de l'oxyde de cérium.

EXEMPLE 2

On a préparé une solution à partir de 103,55 g de nitrate de cérium $Ce(NO_3)_3$, $6H_2O$ et de 5,15 g de nitrate de praséodyme $Pr(NO_3)_3$, $6H_2O$ dissous dans 400 cm³ d'eau et de 0,115 g d'oxyde de terbium $Tb_4O_7$ préalablement dissous dans 11 cm³ d'acide nitrique de normalité 1,4 N. On a ensuite procédé de manière identique à l'exemple 1 en précipitant par 98,1 g d'oxalate d'ammonium.

Après calcination, on a obtenu 41 g d'une solution solide de formule $Ce_{0,95}$ $Pr_{0,0475}$ $Tb_{0,0025}$ $O_2$ de structure type $CeO_2$ de couleur bordeaux foncé caractérisée par les coordonnées trichromatiques L = 36,7, a = 15,9 et b = 8,3.

EXEMPLE 3

On a préparé une solution à partir de 98,1 g de nitrate de cérium $Ce(NO_3)_3$, $6H_2O$, de 5,43 g de nitrate de praséodyme $Pr(NO_3)_3$, $6H_2O$, et de 5,43 g de nitrate de néodyme $Nd(NO_3)_3$, $6H_2O$ que l'on a dissout dans 400 cm³ d'eau. On a procédé ensuite selon l'exemple 1 en précipitant par 98 g d'oxalate d'ammonium.

Après calcination, on a obtenu une poudre de structure type $CeO_2$, de coloration rouge violacé et de coordonnées trichromatiques L = 39,6, a = 22,0 et b = 17,3.

EXEMPLE 4

On a préparé une poudre colorée selon l'exemple 1, en mettant en oeuvre 374,2 g de nitrate de cérium $Ce(NO_3)_3$, $6H_2O$ et 40, 8 g de nitrate de praséodyme $Pr(NO_3)_3$, $6H_2O$.

On a obtenu 160 g d'une solution solide de formule $Ce_{0,9}$ $Pr_{0,1}$ $O_2$ de structure type $CeO_2$.

La coloration est bordeaux foncé caractérisée par les coordonnées trichromatiques L = 34,0, a = 20,6 et b = 11,3.

Application à l'émaillage

## EXEMPLE 5

Dans un bol de broyeur à billes en agate, on met 40 g d'une fritte du commerce (FERRO F.87), 2,5 g des pigments préparés selon les exemples 1 ou 4, 0,12 g de gomme arabique et 50 cm³ d'eau. Le mélange est broyé pendant 30 minutes et tamisé à 100 microns. La barbottine obtenue est pulvérisée au moyen d'un pistolet à air comprimé sur un carreau de faïence VILLEROY et BOCH de dimensions 5 x 5 cm. On dépose ainsi environ 3 g de barbottine par carreau. Le carreau est alors cuit à 950°C pendant 30 minutes. Après refroidissement, on obtient des carreaux de couleur rouge dont les coordonnées trichromatiques en fonction des poudres utilisées sont données au tableau suivant:

| Pigment | L | a | b |
|---------|------|------|------|
| Ex. 1 | 36,1 | 31 | 25,5 |
| Ex. 4 | 33,8 | 24,7 | 20,5 |

## EXEMPLE 6

On a procédé à un émaillage selon l'exemple 5 mais en utilisant une fritte haute température (couverte AG de FERRO ou GTO de CERADEL), et un carreau fabriqué à partir de pâte de porcelaine.

La mise en œuvre est identique à celle de l'exemple 4 à la différence que les carreaux sont cuits à 1250°C. Les résultats de l'émaillage sont donnés au tableau suivant:

| Pigment | L | a | b |
|---------|------|------|------|
| Ex. 1 | 60,5 | 21 | 16,2 |
| Ex. 2 | 42,5 | 28,1 | 32,7 |
| Ex. 3 | 66,1 | 18 | 33,5 |
| Ex. 4 | 61 | 20,5 | 35 |

## EXEMPLE 7

On a mis en œuvre un pigment de cérium et de praséodyme contenant du cuivre comme élément de transition. On a préparé un précurseur de $Ce_{0,95} Pr_{0,05} O_2$ en procédant selon l'exemple 1 et en calcinant l'oxalate mixte obtenu à la précipitation à 580°C. A 10 g de précurseur on a ajouté 0,54 g de chlorure $CuCl_2$. On a calciné le mélange ainsi obtenu 18 heures à 1400°C. On a obtenu une poudre de coloration marron foncé qui mise en œuvre en émaillage selon les exemples 5 et 6 a permis l'obtention de carreaux d'une coloration plus foncée que celle obtenue avec les pigments sans cuivre. Les coordonnées trichromatiques sont données au tableau suivant:

| | Poudre | Carreau 950°C | Carreau 1250°C |
|---|--------|---------------|----------------|
| L | 35,6 | 29,5 | 43,2 |
| a | 15,4 | 17,1 | 26,8 |
| b | 8,5 | 8,8 | 31,0 |

## EXEMPLE 8

On a procédé selon l'exemple 7, mais en ajoutant au précurseur $Ce_{0,95} Pr_{0,05} O_2$ d'une part 0,2 g et d'autre part 0,784 g de sulfate $CuSO_4, 5H_2O$, correspondant à des concentrations exprimées en nombre d'atomes de cuivre par rapport au nombre d'atomes de terres rares de 0,5 et 2% respectivement. On a calciné les mélanges et mis en œuvre les émaillages en procédant selon l'exemple 7. Les colorations plus foncées obtenues sont illustrées par le tableau suivant:

9

| Taux de Cu | | Poudre | Carreau 950°C | Carreau 1250°C |
|---|---|---|---|---|
| 0,5% | L | 37,6 | 25,1 | 47,6 |
| | a | 17,8 | 30,1 | 29,8 |
| | b | 10,9 | 23,4 | 38,7 |
| 2% | L | 35,2 | 20,8 | 41,2 |
| | a | 13,9 | 22,9 | 26,4 |
| | b | 7,9 | 15,7 | 33,1 |

On constate ainsi l'intérêt du cuivre pour l'obtention de colorations plus foncées par rapport aux poudres telles quelles, sans que la mise en œuvre de quantités importantes de cuivre soit nécessire.

Application dans le domaine de la cosmétique

<u>EXEMPLE 9</u>

Cet exemple illustre la mise en œuvre du pigment synthétisé dans l'exemple 1 dans la préparation d'un fond de teint du type émulsion huile dans eau.

On chauffe à 75°C ± 5°C une phase lipophile constituée de:
– 15 g d'huile de vaseline
– 4 g de mono- et distéarate de glycérol (ARLACEL 165)
– 1 g d'alcool cétylique

On disperse sous agitation 1 g de pigment dans la phase lipophile et 3 g d'oxyde de titane.

On ajoute sous agitation une phase hydrophile chauffée à 75°C et contenant:
– 3 g de propylèneglycol
– 0,05 g de para-oxybenzoate de méthyle
– 0,1 g de para-oxybenzoate de propyle
– 20 g d'un sol aqueux à 1% de gomme Xanthane (Rhodopol 23 SC)
– q.s.p. 100 g d'eau distillée

On maintient, sous agitation, pendant le refroidissement jusqu'à température ambiante.

On obtient une émulsion teintée en rose.

## Revendications

1. Pigments répondant à la formule:

$$Ce_{1-x-y} Pr_x A_y O_z \quad (I)$$

dans laquelle:

A est au moins un élément qui peut être:
– un élément terre rare $A_1$ choisi dans le groupe constitué par les lanthanides et l'yttrium et/ou
– un élément de transition $A_2$ choisi dans le groupe formé par les éléments appartenant aux colonnes 1b, 2b, 3b, 4b, 5b, 6b, 7b, 8 de la classification périodique
– x étant un nombre tel que $0,001 \leq x \leq 0,10$
– y étant un nombre tel que $0 \leq y \leq 0,1$ et
– z étant égal à

$$\frac{4 - (4-n)y}{2}$$

– n étand le degré d'oxydation de A
ayant une structure cristallographique unique isotype de celle de $CeO_2$ et présentant un seuil de réflectance d'au moins 580 nm.

2. Pigments selon la revendication 1, caractérisés par le fait que $A_1$ est choisi parmi les éléments terres rares suivants: terbium, néodyme, erbium, dysprosium et europium.

3. Pigments selon l'une des revendications 1 ou 2, caractérisés par le fait que $A_2$ est choisi parmi les éléments de transition suivants: titane, vanadium, chrome, manganèse, cuivre, fer, cobalt, nickel et molybdène.

4. Pigments selon l'une des revendications 1 à 3, caractérisés par le fait que x est inférieur ou égal à 0,06.

5. Pigments selon l'une des revendications 1 à 4, caractérisés par le fait que y est inférieur ou égal à

10

0,05.

6. Pigments selon la revendication 1 de formule:

$Ce_{1-x} Pr_x O_2$

x étant tel que défini à la revendication 1.

7. Pigments selon la revendication 1, caractérisés par le fait que l'élément $A_2$ est le cuivre.

8. Procédé de préparation de pigments décrits dans l'une des revendications 1 à 7, caractérisé par le fait qu'il consiste:

a) à effectuer une co-précipitation de composés de cérium, de praséodyme et éventuellement de l'élément $A_1$, à partir d'une solution aqueuse de sels solubles des éléments précités,

b) à séparer le co-précipité obtenu,

c) à ajouter éventuellement un composé de l'élément $A_1$ et/ou un composé de l'élément $A_2$ sous forme solide, au co-précipité obtenu,

d) à calciner le mélange obtenu à une température d'au moins 800°C.

9. Procédé selon la revendication 8, caractérisé par le fait que la solution aqueuse de sels solubles du cérium, du praséodyme et éventuellement de l'élément $A_1$ peut être une solution de nitrates, chlorures et/ou sulfates desdits éléments.

10. Procédé selon l'une des revendications 8 et 9, caractérisé par le fait que l'on co-précipite lesdits éléments sous forme d'hydroxydes, oxalates ou carbonates.

11. Procédé selon la revendication 8, caractérisé par le fait que l'on effectue une co-précipitation des hydroxydes par mélange de la solution aqueuse des sels solubles desdits éléments avec une solution basique.

12. Procédé selon la revendication 11, caractérisé par le fait que le pH du milieu réactionnel est de 6 à 8,5.

13. Procédé selon l'une des revendications 11 et 12, caractérisé par le fait que la température du milieu réactionnel est comprise entre 50 et 70°C.

14. Procédé selon la revendication 8, caractérisé par le fait que l'on effectue une co-précipitation des oxalates par mélange de la solution aqueuse des sels solubles desdits éléments avec l'acide oxalique ou ses sels.

15. Procédé selon la revendication 14, caractérisé par le fait que l'agent précipitant est l'oxalate d'ammonium.

16. Procédé selon l'une des revendications 14 et 15, caractérisé par le fait que le pH du milieu réactionnel est de 1 à 3.

17. Procédé selon l'une des revendications 14 à 16, caractérisé par le fait que la température du milieu réactionnel est comprise entre 80 et 90°C.

18. Procédé selon l'une des revendications 8 à 17, caractérisé par le fait que l'on opère éventuellement un mûrissement avant l'opération de filtration à une température comprise entre 10 et 95°C pendant un temps pouvant aller de 30 minutes à 2 heures.

19. Procédé selon l'une des revendications 8 à 18, caractérisé par le fait que l'on effectue une séparation du co-précipité par filtration.

20. Procédé selon la revendication 19, caractérisé par le fait que l'on effectue éventuellement un lavage à l'eau du gâteau de filtration.

21. Procédé selon l'une des revendications 8 à 20, caractérisé par le fait que l'on effectue une étape de séchage à une température comprise entre 100 et 550°C.

22. Procédé selon la revendication 14, caractérisé par le fait que l'on effectue une pré-calcination à une température comprise entre 350 et 700°C.

23. Procédé selon l'une des revendications 8 à 22, caractérisé par le fait que l'on mélange avant calcination le co-précipité séparé avec un oxyde de l'élément $A_1$ ou un sel de cet élément décomposable dans les conditions de la calcination.

24. Procédé selon l'une des revendications 8 à 23, caractérisé par le fait que l'on mélange avant calcination le co-précipité séparé avec un oxyde de l'élément $A_2$ ou un sel de cet élément décomposable dans les conditions de calcination.

25. Procédé selon l'une des revendications 8 à 24, caractérisé par le fait que l'on ajoute un fondant avant l'opération de calcination.

26. Procédé selon la revendication 25, caractérisé par le fait que le fondant est le fluorure de sodium, le chlorure de sodium ou leurs mélanges.

27. Procédé selon l'une des revendications 8 à 26, caractérisé par le fait que la calcination est effectuée à une température de 1000 à 1400°C.

28. Procédé selon l'une des revendications 8 à 27, caractérisé par le fait que le produit obtenu est broyé ensuite à une taille de particules de 0,1 à 10 μm.

29. Utilisation des pigments décrits dans l'une des revendications 1 à 7 dans l'industrie des peintures et lasures, des matières plastiques, du papier, des encres d'imprimerie, des cosmétiques, des textiles et dans l'industrie de la céramique.

30. Utilisation des pigments obtenus selon le procédé décrit dans l'une des revendications 8 à 28 dans l'industrie des peintures et lasures, des matières plastiques, du papier, des encres d'imprimerie, des cosmétiques, des textiles et dans l'industrie de la céramique.

31. Utilisation des pigments décrits dans l'une des revendications 1 à 7 dans les céramiques pour des températures de mise en œuvre allant jusqu'à 1250°C.

32. Utilisation des pigments obtenus selon le procédé décrit dans l'une des revendications 8 à 28 dans les céramiques pour des températures de mises en œuvre allant jusqu'à 1250°C.

33. Utilisation des pigments décrits dans l'une des revendications 1 à 7 en cosmétique, dans les vernis à ongles et dans les fards.

34. Utilisation selon la revendication 33 caractérisée par le fait que les pigments sont mis en oeuvre dans les rouges à lèvres, dans les fards secs ou gras à joues ou à paupières, dans les fonds de teint.

## Patentansprüche

1. Pigmente gemäß der Formel
$Ce_{1-x-y} Pr_x A_y O_z$ (I)
in der
A wenigstens eines der folgenden Elemente ist,
– ein Seltenerdelement $A_1$ ausgewählt aus der Gruppe bestehend aus den Lanthaniden und Yttrium und/oder
– ein Übergangselement $A_2$ ausgewählt aus der Gruppe bestehend aus den Elementen der Gruppen 1b, 2b, 3b, 4b, 5b, 6b, 7b und 8 des Periodensystems
– x ein Wert ist, für den gilt $0{,}001 \leq x \leq 0{,}10$
– y ein Wert ist, für den gilt $0 \leq y \leq 0{,}1$ und
– z gleich

$$\frac{4 - (4-n)y}{2}$$

ist
– n den Oxidationsgrad von A angibt
die eine einzige isotype kristallografische $CeO_2$-Struktur haben und eine Reflektanzschwelle von wenigstens 580 nm aufweisen.

2. Pigmente gemäß Anspruch 1, dadurch gekennzeichnet, daß $A_1$ ausgewählt wird aus den folgenden Seltenerdelementen: Terbium, Neodym, Erbium, Dysprosium und Europium.

3. Pigmente gemäß einem der Ansprüche 1 und 2, dadurch gekennzeichnet, daß $A_2$ ausgewählt wird aus den folgenden Übergangselementen: Titan, Vanadium, Chrom, Mangan, Kupfer, Eisen, Kobalt, Nickel und Molybdän.

4. Pigmente gemäß einem der Ansprüche 1 bis 2, dadurch gekennzeichnet, daß x kleiner oder gleich 0,06 ist.

5. Pigmente gemäß einem der Ansprüche 1 bis 4, dadurch gekennzeichnet, daß y kleiner oder gleich 0,05 ist.

6. Pigmente gemäß Anspruch 1, mit der Formel:
$Ce_{1-x} Pr_x O_2$
wobei x der Definition von Anspruch 1 entspricht.

7. Pigmente gemäß Anspruch 1, dadurch gekennzeichnet, daß das Element $A_2$ Kupfer ist.

8. Verfahren zur Herstellung der in einem der Ansprüche 1 bis 7 beschriebenen Pigmente, dadurch gekennzeichnet, daß es umfaßt:
a) das Ausführen einer Cofällung der Verbindung des Cers, Praseodyms und gegebenenfalls des Elements $A_1$, ausgehend von einer wässrigen Lösung löslicher Salze der vorgenannten Elemente,
b) das Abtrennen des erhaltenen Copräzipitats,
c) gegebenenfalls das Hinzufügen einer Verbindung des Elements $A_1$ und/oder einer Verbindung des Elements $A_2$ in fester Form, zu dem erhaltenen Copräzipitat,
d) das Kalzinieren des erhaltenen Gemischs bei einer Temperatur von wenigstens 800°C.

9. Verfahren gemäß Anspruch 8, dadurch gekennzeichnet, daß die wässrige Lösung der löslichen Salze des Cers, des Praseodyms und gegebenenfalls des Elements $A_1$ eine Lösung von Nitraten, Chloriden und/oder Sulfaten der genannten Elemente ist.

10. Verfahren gemäß einem der Ansprüche 8 oder 9, dadurch gekennzeichnet, daß man die genannten Elemente in Form von Hydroxiden, Oxalaten oder Carbonaten gemeinsam fällt.

11. Verfahren nach Anspruch 8, dadurch gekennzeichnet, daß man eine Copräzipitation von Hydroxiden durch Mischen der wässrigen Lösung der löslichen Salze der genannten Elemente mit einer basischen Lösung bewirkt.

12. Verfahren nach Anspruch 11, dadurch gekennzeichnet, daß der pH des Reaktionsmilieus zwischen 6 und 8,5 liegt.

13. Verfahren nach einem der Ansprüche 11 oder 12, dadurch gekennzeichnet, daß die Temperatur des Reaktionsmilieus zwischen 50 und 70°C liegt.

14. Verfahren nach Anspruch 8, dadurch gekennzeichnet, daß man eine Copräzipitation der Oxalate durch Mischen der wässrigen Lösung der löslichen Salze der genannten Elemente mit Oxalsäure oder ihren Salzen bewirkt.

15. Verfahren nach Anspruch 14, dadurch gekennzeichnet, daß das Fällungsagens Ammoniumoxalat ist.

16. Verfahren nach einem der Ansprüche 14 oder 15, dadurch gekennzeichnet, daß der pH des Reaktionsmilieus 1 bis 3 beträgt.

17. Verfahren nach einem der Ansprüche 14 bis 16, dadurch gekennzeichnet, daß die Temperatur des Reaktionsmilieus 80 bis 90°C beträgt.

18. Verfahren nach einem der Ansprüche 8 bis 17, dadurch gekennzeichnet, daß man gegebenenfalls eine Reifung vor dem Filtriervorgang durchführt bei einer Temperatur zwischen 10 und 95°C für eine Zeitdauer, die von 30 Minuten bis 2 Stunden betragen kann.

19. Verfahren nach einem der Ansprüche 8 bis 18, dadurch gekennzeichnet, daß man eine Abtrennung des Copräzipitats durch Filtrierung vornimmt.

20. Verfahren nach Anspruch 19, dadurch gekennzeichnet, daß man gegebenenfalls eine Waschung des Filterkuchens mit Wasser ausführt.

21. Verfahren nach einem der Ansprüche 8 bis 20, dadurch gekennzeichnet, daß man einen Trocknungsschritt bei einer Temperatur von 100 bis 550°C durchführt.

22. Verfahren nach Anspruch 14, dadurch gekennzeichnet, daß man eine Präkalzinierung bei einer Temperatur von 350 bis 700°C ausführt.

23. Verfahren nach einem der Ansprüche 8 bis 22, dadurch gekennnzeichnet, daß man vor der Kalzinierung das abgetrennte Copräzipitat mit einem Oxid des Elements $A_1$ oder einem Salz dieses Elements mischt, das unter den Bedingungen der Kalzinierung zersetzbar ist.

24. Verfahren nach einem der Ansprüche 8 bis 23, dadurch gekennzeichnet, daß man vor der Kalzinierung das abgetrennte Copräzipitat mit einem Oxid des Elements $A_2$ oder einem Salz dieses Elements mischt, das unter den Bedingungen der Kalzinierung zersetzbar ist.

25. Verfahren nach einem der Ansprüche 8 bis 24, dadurch gekennzeichnet, daß man vor dem Kalzinierungsschritt ein Flußmittel zugibt.

26. Verfahren nach Anspruch 25, dadurch gekennzeichnet, daß das Flußmittel Natriumfluorid, Natriumchlorid oder deren Mischungen ist.

27. Verfahren nach einem der Ansprüche 8 bis 26, dadurch gekennzeichnet, daß die Kalzinierung bei einer Temperatur von 1000 bis 1400°C ausgeführt wird.

28. Verfahren nach einem der Ansprüche 8 bis 27, dadurch gekennzeichnet, daß das erhaltene Produkt anschließend auf eine Partikelgröße von 0,1 bis 10 µm gemahlen wird.

29. Verwendung der in einem der Ansprüche 1 bis 7 beschriebenen Pigmente in der Farben- und Lasurenindustrie, der Kunststoffindustrie, der Papierindustrie, der Druckfarbenindustrie, der Kosmetikindustrie, der Textilindustrie und der Keramikindustrie.

30. Verwendung der in einem Verfahren gemäß einem der Ansprüche 8 bis 28 erhaltenen Pigmente in der Farben- und Lasurenindustrie, der Kunststoffindustrie, der Papierindustrie, der Druckfarbenindustrie, der Kosmetikindustrie, der Textilindustrie und der Keramikindustrie.

31. Verwendung der Pigmente, die in einem der Ansprüche 1 bis 7 beschrieben sind, in Keramik für Anwendungstemperaturen bis zu 1250°C.

32. Verwendung der in einem Verfahren gemäß einem der Ansprüche 8 bis 28 beschriebenen Pigmente in Keramik für Anwendungstmeperaturen bis zu 1250°C.

33. Verwendung der in einem der Ansprüche 1 bis 7 beschriebenen Pigmente in der Kosmetik, in Nagellacken und Schminken.

34. Verwendung gemäß Anspruch 33, dadurch gekennzeichnet, daß die Pigmente in Lippenstiften, trockenen oder fettigen Schminken für Wangen oder Lider oder in Make-up eingesetzt werden.

## Claims

1. Pigments corresponding to the formula:

$$Ce_{1-x-y} Pr_x A_y O_z \quad (I)$$

in which:

A is at least one element which may be:
- a rare-earth element $A_1$ chosen from the group consisting of the lanthanides and yttrium and/or
- a transition element $A_2$ chosen from the group consisting of the elements belonging to columns 1b, 2b, 3b, 4b, 5b, 6b, 7b and 8 of the Periodic Classification
- x being a number such that $0.001 \leq x \leq 0.10$
- y being a number such that $0 \leq y \leq 0.1$, and
- z being equal to

$$\underline{4 - (4-n)y}$$
$$2$$

— n being the oxidation state of A

having a single crystallographic structure isotypic with that of $CeO_2$ and having a reflectance threshold of at least 580 nm.

2. Pigments according to Claim 1, characterized in that $A_1$ is chosen from the following rare-earth elements: terbium, neodymium, erbium, dysprosium and europium.

3. Pigments according to either of Claims 1 and 2, characterized in that $A_2$ is chosen from the following transition elements: titanium, vanadium, chromium, manganese, copper, iron, cobalt, nickel and molybdenum.

4. Pigments according to one of Claims 1 to 3, characterized in that x is smaller than or equal to 0.06.

5. Pigments according to one of Claims 1 to 4, characterized in that y is smaller than or equal to 0.05.

6. Pigments according to Claim 1, of formula:

$Ce_{1-x} Pr_x O_2$

x being such as defined in Claim 1.

7. Pigments according to Claim 1, characterized in that the element $A_2$ is copper.

8. Process for preparing pigments described in one of Claims 1 to 7, characterized in that it consists:

a) in performing a coprecipitation of compounds of cerium, of praseodymium and, if desired, of the element $A_1$, from an aqueous solution of soluble salts of the abovementioned elements,

b) in separating the coprecipitate obtained,

c) in adding, if desired, a compound of the element $A_1$ and/or a compound of the element $A_2$ in solid form to the coprecipitate obtained,

d) in calcining the mixture obtained at a temperature of at least 800°C.

9. Process according to Claim 8, characterized in that the aqueous solution of soluble salts of cerium, of praseodymium and, if desired, of the element $A_1$ may be a solution of nitrates, chlorides and/or sulphates of the said elements.

10. Process according to either of Claims 8 and 9, characterized in that the said elements are coprecipitated in the form of hydroxides, oxalates or carbonates.

11. Process according to Claim 8, characterized in that a coprecipitation of hydroxides is performed by mixing the aqueous solution of the soluble salts of the said elements with a basic solution.

12. Process according to Claim 11, characterized in that the pH of the reaction medium is from 6 to 8.5.

13. Process according to either of Claims 11 and 12, characterized in that the temperature of the reaction medium is between 50 and 70°C.

14. Process according to Claim 8, characterized in that a coprecipitation of oxalates is performed by mixing the aqueous solution of the soluble salts of the said elements with oxalic acid or its salts.

15. Process according to Claim 14, characterized in that the precipitating agent is ammonium oxalate.

16. Process according to either of Claims 14 and 15 , characterized in that the pH of the reaction medium is from 1 to 3.

17. Process according to one of Claims 14 to 16 , characterized in that the temperature of the reaction medium is between 80 and 90°C.

18. Process according to one of Claims 8 to 17, characterized in that an aging operation is carried out if desired before the filtration operation at a temperature of between 10 and 95°C for a time which can range from 30 minutes to 2 hours.

19. Process according to one of Claims 8 to 18, characterized in that a separation of the coprecipitate is performed by filtering.

20. Process according to Claim 19, characterized in that the filter cake is washed with water if desired.

21. Process according to one of Claims 8 to 20, characterized in that a drying step is performed at a temperature of between 100 and 550°C.

22. Process according to Claim 14, characterized in that a precalcination is performed at a temperature of between 350 and 700°C.

23. Process according to one of Claims 8 to 22, characterized in that, before calcination, the separated coprecipitate is mixed with an oxide of the element $A_1$ or a salt of this element capable of decomposing under the conditions of the calcination.

24. Process according to one of Claims 8 to 23, characterized in that, before calcination, the separated coprecipitate is mixed with an oxide of the element $A_2$ or a salt of this element which is capable of decomposing under the conditions of calcination.

25. Process according to one of Claims 8 to 24, characterized in that a flux is added before the calcining operation.

26. Process according to Claim 25, characterized in that the flux is sodium fluoride, sodium chloride or mixtures thereof.

27. Process according to one of Claims 8 to 26, characterized in that the calcination is performed at a temperature of 1000 to 1400°C.

28. Process according to one of Claims 8 to 27, characterized in that the product obtained is then milled to a particle size of 0.1 to 10 μm.

29. Use of the pigments described in one of Claims 1 to 7 in the paint and glaze industry, plastics, paper, printing inks, cosmetics, textiles and in the ceramics industry.

30. Use of the pigments obtained according to the process described in one of Claims 8 to 28 in the paint and glaze industry, plastics, paper, printing inks, cosmetics, textiles and in the ceramics industry.

31. Use of the pigments described in one of Claims 1 to 7 in ceramics for application temperatures ranging up to 1250°C.

32. Use of the pigments obtained according to the process described in one of Claims 8 to 28 in ceramics for application temperatures ranging up to 1250°C.

33. Use of the pigments described in one of Claims 1 to 7 in cosmetics, in nail varnishes and in rouges.

34. Use according to Claim 33, characterized in that the pigments are used in lipsticks, in dry or fatty rouges for cheeks or for eyelids, and in makeup foundations.